# EUROPÄISCHE PATENTSCHRIFT

(11) **EP 3 123 688 B1**
(45) Veröffentlichungstag und Bekanntmachung des Hinweises auf die Patenterteilung: **02.05.2018**
(21) Anmeldenummer: 15713413.1
(22) Anmeldetag: 23.03.2015
(51) Int. Cl.: H04L 29/06, H04W 12/06, H04L 29/08

(54) **MEDIZINTECHNISCHES GERÄT MIT NETZWERKKONTROLLEINRICHTUNG**
MEDICAL EQUIPMENT COMPRISING NETWORK CONTROL DEVICE
APPAREIL MÉDICAL ÉQUIPÉ D'UN MOYEN DE CONTRÔLE DE RÉSEAU

(30) Priorität: 24.03.2014 DE 202014002535 U
(43) Veröffentlichungstag der Anmeldung: 01.02.2017
(73) Patentinhaber: Fresenius Medical Care Deutschland GmbH, 61352 Bad Homburg (DE)
(72) Erfinder: KLEMENS, Matthias, 97456 Dittelbrunn (DE); SIEBERT, Jochen, 97618 Strahlungen (DE)
(74) Vertreter: Lindemann, Robert
(86) Internationale Anmeldenummer: PCT/EP2015/056080
(87) Internationale Veröffentlichungsnummer: WO 2015/144626

(56) Entgegenhaltungen:
- DE-A1-102008 000 895
- US-A1- 2008 126 729

## Beschreibung

### Technisches Gebiet

Die Erfindung betrifft das Gebiet von medizintechnischen Geräten, insbesondere in Blutbehandlungsgeräten wie Dialysegeräte.

### Hintergrund

Blutbehandlungsgeräte sind medizintechnische Geräte, bei denen das Blut eines Patienten oftmals extrakorporal behandelt wird. Beispielsweise können dem Blut Medikamente zugesetzt oder ihm Blutbestandteile entzogen werden, es kann erwärmt oder gekühlt werden. Zu diesem Zweck wird das Blut oftmals außerhalb des Körpers mit einer Blutpumpe gepumpt, behandelt und dem Körper des Patienten wieder zurückgegeben. Ein solcher Blutkreislauf heißt extrakorporaler Blutkreislauf.

Im Folgenden soll die Erfindung anhand des Beispiels Dialysegerät als Ausführungsform eines medizintechnischen Geräts erklärt werden.

Weitere Blutbehandlungsgeräte sind beispielsweise Geräte zur Unterstützung der Herz-Lungentätigkeit, wie Blutoxygenatoren, oder Geräte zur Unterstützung der Leberfunktion, die Bluttoxine durch Adsorption aus dem Blut entfernen. Oftmals kombinieren Geräte zur Unterstützung der Leberfunktion Dialyseverfahren und Adsorptionsverfahren in einem Gerät, wie beispielsweise das Gerät Prometheus der Anmelderin.

Dialysegeräte sind Blutbehandlungsgeräte, bei denen das Blut eines Patienten über eine Blutleitung einer Blutbehandlungskomponente zugeführt, durch die Blutbehandlungskomponente behandelt und über die Blutleitung, die in einen arteriellen und einen venösen Zweig aufgeteilt werden kann, wieder an den Patienten zurückgegeben wird. Beispiele für solche Blutbehandlungsgeräte sind insbesondere Hämodialysegeräte. Ein solches Blutbehandlungsgerät ist Gegenstand der DE 198 49 787 C1 der Anmelderin, deren Inhalt hiermit vollumfänglich im Offenbarungsgehalt der vorliegenden Anmeldung einbezogen wird.

Die Dialyse ist ein Verfahren zur Blutreinigung von Patienten mit akuter oder chronischer Niereninsuffizienz. Grundsätzlich unterscheidet man hierbei zwischen Verfahren mit einem extrakorporalen Blutkreislauf, wie der Hämodialyse, der Hämofiltration oder der Hämodiafiltration und der Peritonealdialyse, die keinen extrakorporalen Blutkreislauf aufweist.

Das Blut wird bei der Hämodialyse in einem extrakorporalen Kreislauf durch die Blutkammer eines Dialysators geleitet, die über eine semipermeable Membran von einer Dialysierflüssigkeitskammer getrennt ist. Die Dialysierflüssigkeitskammer wird von einer die Blutelektrolyte in einer bestimmten Konzentration enthaltenen Dialysierflüssigkeit durchströmt. Die Stoffkonzentration der Blutelektrolyte in der Dialysierflüssigkeit entspricht dabei der Konzentration im Blut eines Gesunden. Während der Behandlung werden das Blut des Patienten und die Dialysierflüssigkeit an beiden Seiten der semipermeablen Membran im Allgemeinen im Gegenstrom mit einer vorgegebenen Flussrate vorbeigeführt. Die harnpflichtigen Stoffe diffundieren durch die Membran von der Blutkammer in die Kammer für Dialysierflüssigkeit, während gleichzeitig im Blut und in der Dialysierflüssigkeit vorhandene Elektrolyte von der Kammer höherer Konzentration zur Kammer niedrigerer Konzentration diffundieren. Wird an der Dialysemembran ein Druckgradient von der Blutseite zur Dialysatseite aufgebaut, beispielsweise durch eine Pumpe, die flussabwärts des Dialysefilters auf der Dialysatseite Dialysat aus dem Dialysatkreislauf entzieht, tritt Wasser aus dem Patientenblut über die Dialysemembran in den Dialysatkreislauf über. Dieser Vorgang der Ultrafiltration führt zu einer gewünschten Entwässerung des Patientenbluts.

Bei der Hämofiltration wird dem Patientenblut durch Anlegen eines Transmembrandrucks im Dialysator Ultrafiltrat entzogen, ohne dass Dialysierflüssigkeit auf der dem Patientenblut gegenüber liegenden Seite der Membran des Dialysators vorbeigeführt wird. Zusätzlich kann dem Patientenblut eine sterile und pyrogenfreie Substituatslösung zugesetzt werden. Je nachdem, ob diese Substituatslösung stromaufwärts des Dialysators zugesetzt wird oder stromabwärts, spricht man von Prä- oder Postdilution. Der Stoffaustausch erfolgt bei der Hämofiltration konvektiv.

Die Hämodiafiltration kombiniert die Verfahren der Hämodialyse und der Hämofiltration. Es findet sowohl ein diffusiver Stoffaustausch zwischen Patientenblut und Dialysierflüssigkeit über die semipermeable Membran eines Dialysators statt, als auch eine Abfiltrierung von Plasmawasser durch einen Druckgradienten an der Membran des Dialysators.

Die Verfahren der Hämodialyse, der Hämofiltration und der Hämodiafiltration werden in der Regel mit automatischen Hämodialysegeräten durchgeführt, wie sie beispielsweise von der Anmelderin unter der Bezeichnung 5008 vertrieben werden.

Die Plasmapherese ist ein Blutbehandlungsverfahren, bei dem das Patientenblut in das Blutplasma und seine korpuskularen Bestandteile (Zellen) aufgetrennt wird. Das abgetrennte Blutplasma wird gereinigt oder durch eine Substitutionslösung ersetzt und das gereinigte Blutplasma oder die Substitutionslösung dem Patienten zurückgegeben.

Bei der Peritonealdialyse wird die Bauchhöhle eines Patienten über einen durch die Bauchdecke geführten Katheter mit einer Dialyseflüssigkeit befüllt, die ein Konzentrationsgefälle gegenüber den körpereigenen Flüssigkeiten aufweist. Über das als Membran wirkende Bauchfell (Peritoneum) treten die im Körper vorliegenden Giftstoffe in die Bauchhöhle über. Nach einigen Stunden wird die sich in der Bauchhöhle des Patienten befindliche, nunmehr verbrauchte Dialyse-flüssigkeit ausgetauscht. Durch osmotische Vorgänge kann Wasser aus dem Blut des Patienten über das Bauchfell in die Dialyseflüssigkeit übertreten und den Patienten somit entwässern.

Das Verfahren zur Peritonealdialyse wird in der Regel mit Hilfe von automatischen Peritonealdialysegeräten, die ebenfalls medizintechnische Geräte sind, und beispielsweise von der Anmelderin unter der Bezeichnung sleep.safe vertrieben werden, durchgeführt.

Dialysegeräte, als Beispiel für komplexe medizintechnische Geräte, haben umfangreiche Funktionen. Um diese Funktionen zu steuern, sind medizinische Fluidmanagementgeräte wie Dialysegeräte mit zumindest einer Steuervorrichtung ausgerüstet. Diese können als CPU (central processing unit) oder Mikrokontroller ausgeführt sein, die von Softwareprogrammen programmiert werden.

Zum Datenaustausch sind medizintechnische Geräte oftmals mit einer Schnittstelle zum Datenaustausch mit einem Netzwerk ausgerüstet. Eine solche Schnittstelle kann kabelgebunden, beispielsweise als RJ45 Datenschnittstelle ausgeführt, oder kabellos, beispielsweise als WLAN oder Bluetooth Schnittstelle ausgeführt sein.

Über eine solche Datenschnittstelle können eine Vielzahl von Daten ausgetauscht werden. Beispielsweise kann die Software des medizintechnischen Geräts durch ein sogenanntes Online-Update durch Zugriff auf entfernte Daten aktualisiert werden.

Weiterhin können Behandlungsdaten, wie Einstellungen, Messwerte therapeutischer oder physiologischer Parameter oder auch Patientendaten an ein entferntes Gerät, beispielsweise an einen Server zur Abspeicherung und Verfügbarmachung für Dritte gesendet werden.

Prinzipiell besteht die Möglichkeit, dass ein unberechtigter Datenaustausch über die Schnittstelle zum Datenaustausch mit einem Netzwerk stattfindet. Weiterer Stand der Technik ist in der Patentanmeldung DE102008000895 offenbart.

### Beschreibung

Es ist die Aufgabe der vorliegenden Erfindung, ein medizintechnisches Gerät zu schaffen, welches den Datenaustausch mit einem Netzwerk sicherer gestaltet.

Die Lösung dieser Aufgabe erfolgt erfindungsgemäß durch das medizintechnische Gerät nach Anspruch 1.

Weiterhin wird die Aufgabe durch ein System nach Anspruch 7, sowie einem Verfahren nach Anspruch 9 gelöst.

Vorteilhafte Ausführungsformen sind Gegenstand der Unteransprüche.

Ein medizintechnisches Gerät nach der Lehre der vorliegenden Erfindung umfasst eine Steuerelektronik und eine Schnittstelle zum Datenaustausch mit einem Netzwerk. Als Steuerelektronik wird jede elektronische Schaltung verstanden, mit deren Hilfe die Schnittstelle zum Datenaustausch abhängig vom Vorhandensein einer Authentifiziereinrichtung im medizintechnischen Gerät aktiviert oder deaktiviert werden kann. Eine derartige Steuerelektronik kann insbesondere ein Steuergerät sein, welches als softwareprogrammierbarer Mikrokontroller oder als Prozessor ausgeführt sein kann. Die Steuerelektronik kann aber auch jede sonstige Schaltung sein, die dazu dient, eine Schnittstelle zum Datenaustausch abhängig vom Vorhandensein einer Authentifiziereinrichtung im medizintechnischen Gerät zu aktivieren oder zu deaktivieren.

Eine Authentifiziereinrichtung im Einklang mit der Lehre der vorliegenden Erfindung kann ein Kartenlesegerät sein. Ein solches Kartenlesegerät ist dazu eingerichtet, Daten, die auf sogenannten SmardCards gespeichert sind, einzulesen und gegebenenfalls auch Daten auf SmardCards zu schreiben. Zu diesem Zweck weisen SmardCards zumindest eine Datenschnittstelle auf, welche beispielsweise als elektrische Kontaktflächen, Magnetstreifen oder Antennenkonfigurationen zum Empfang und Aussenden von Funknachrichten ausgeführt sein können.

Zum Speichern von Daten weisen SmardCards eine Datenspeichervorrichtung auf, die wiederbeschreibbar sein kann. Eine solche Datenspeichervorrichtung ist beispielsweise ein nichtflüchtiger, also ohne Energiezufuhr selbsterhaltender, sogenannter Flash EEPROM Speicher. Eine andere Ausführungsform, die Schnittstelle und Speicher kombiniert, ist ein Magnetstreifen.

SmardCards bieten die Möglichkeit, ihren Benutzer eindeutig zu authentifizieren, in dem sie ein individuelles Erkennungsmerkmal abspeichern, welches durch das Kartenlesegerät abrufbar ist. Ein solches Erkennungsmerkmal kann eine beliebige Kombination von Zeichen und Ziffern sein, welche einer einzigen Person zugeordnet ist. Die Zuordnung von Person zu Erkennungsmerkmal kann an zentraler Stelle abgespeichert sein, beispielsweise im medizinischen Gerät.

Um sicher zu gehen, dass eine bestimmte SmardCard auch von der ihr zugeordneten Person benutzt wird, kann mit dem individuellen Erkennungsmerkmal auch eine zweite nur der zugeordneten Person ausgehändigte individuelle Zeichenkette, beispielsweise eine Geheimnummer verknüpft sein. Die mit dem Kartenlesegerät ausgestattete Vorrichtung kann dementsprechend so konfiguriert sein, dass nach Einlesen einer SmardCard der Benutzer aufgefordert wird, die individuelle Zeichenkette in die Vorrichtung einzugeben. Zur Ein- bzw. Ausgabe von Informationen kann eine solche Vorrichtung zweckmäßig mit einem Touchscreendisplay ausgestattet sein.

Durch die Eingabe der richtigen individuellen Zeichenkette authentifiziert sich der Benutzer an der Vorrichtung. Hierfür überprüft die Vorrichtung die Eingabe anhand der ihr bekannt gemachten Zuordnung von individuellem Erkennungsmerkmal und individueller Zeichenkette.

Weitere Authentifiziereinrichtungen können ein Fingerabdruckscanner oder ein Irisscanner sein. Beide Sensoren bringen mit der Messung biometrischer Merkmale personenindividuelle Daten in Erfahrung, die einer einzelnen Person zugeordnet werden können.

Durch die Authentifiziereinrichtung kann die Identität des Benutzers eindeutig bestimmt werden. Soll die Identität beispielsweise aus Datenschutzgründen nicht gespeichert werden, kann in derselben Art und Weise statt der Identität einer Person ein individuelles Erkennungsmerkmal, beispielsweise eine anonyme Zeichenkette verknüpft werden.

Die Identität als auch das individuelle Erkennungsmerkmal können beispielsweise auch mit Berechtigungen verknüpft werden. Eine Berechtigung im Einklang mit der Lehre der vorliegenden Erfindung ist eine vom medizintechnischen Gerät verwertbare Information, welche Aktionen ein authentifizierter Benutzer am medizinischen Gerät durchführen darf und welche nicht. Diese Information wird mit der Identität und/oder mit dem individuellen Erkennungsmerkmal verknüpft und dem medizintechnischen Gerät beispielsweise durch Hinterlegung in einem Datenspeicher bekannt gemacht. Eine Berechtigung kann auch zusammen mit der Identität oder dem individuellen Erkennungsmerkmal auf einer SmardCard des Benutzers gespeichert sein.

Authentifizierungseinrichtungen sind oftmals keine Standardausstattung medizintechnischer Geräte. Sie stellen oftmals eigenständige Module dar, mit denen medizintechnische Geräte nachgerüstet werden und in diese durch Montage integriert werden.

Datenschnittstellen hingegen sind häufig Teil eine Hauptplatine und somit häufig standardmäßiger Teil eines medizintechnischen Geräts.

Eine stets aktivierte Datenschnittstelle könnte zu einem unberechtigten Datenaustausch mit einem Netzwerk benutzt werden. Eine Berechtigung zum Datenaustausch kann beispielsweise die Authentifizierung des Benutzers verlangen.

Sind medizintechnische Geräte mit einer Authentifizierungseinrichtung ausgestattet, so sind die medizintechnischen Geräte oftmals so eingerichtet, dass deren Funktion von der Authentifizierung des Benutzers abhängt.

In einer Ausführungsform der Erfindung wird die Schnittstelle zum Datenaustausch mit einem Netzwerk abhängig vom Vorhandensein einer Authentifiziereinrichtung im medizintechnischen Gerät aktiviert oder deaktiviert.

Hierzu kann die Steuerelektronik des medizintechnischen Geräts eine Erkennungseinrichtung aufweisen, die so konfiguriert ist, dass die Ausrüstung des medizintechnischen Geräts mit einer Authentifiziereinrichtung anhand der Überprüfung zumindest eines Signals erkannt wird, welches durch die Ausrüstung mit der Authentifiziereinrichtung erzeugt wird.

Ein solches Signal kann beispielsweise ein elektrisches Signal sein, beispielsweise eine elektrische Spannung, die an der Authentifiziereinrichtung erzeugt wird, wenn sie in das medizintechnische Gerät eingebaut ist. So kann beispielsweise der Einbau eines Kartenlesegeräts derart geschehen, in dem dieses in einem vorbereiteten Einschub des medizintechnischen Geräts montiert wird, und dabei der elektrische Kontakt der Steuer- und Versorgungssignale zwischen medizinischem Gerät und Kartenlesegerät durch eine Kontaktleiste hergestellt wird.

Eine solche Kontaktleiste kann beispielsweise eine Steckkartenverbindung sein. Wird das Kartenlesegerät über die Steckkartenverbindung mit dem medizintechnischen Gerät elektrisch verbunden, kann beispielsweise ein Kontakt bzw. eine Kontaktfläche der Steckkartenverbindung durch eine entsprechende elektrische Verbindung im Kartenlesegerät mit einer vom medizinischen Gerät gelieferten Spannung verbunden werden. Wird dieser Pol von der Erkennungseinrichtung überwacht, kann darauf geschlossen werden, dass ein Kartenlesegerät eingebaut ist, wenn die Spannung einen vorher definierten Wert annimmt. In diesem Fall kann die Steuerelektronik die Schnittstelle zum Datenaustausch mit einem Netzwerk aktivieren.

Das Aktivieren der Schnittstelle zum Datenaustausch mit einem Netzwerk bedeutet im Einklang mit der Lehre der vorliegenden Erfindung beispielsweise das Anstellen der Spannungsversorgung oder das Herstellen einer Verbindung von Signalleitungen von der Schnittstelle zu der datenverarbeitenden Vorrichtung, beispielsweise der Steuerelektronik.

In einer weiteren Ausführungsform wird zusätzlich zur Überprüfung des Vorhandenseins einer Authentifiziereinrichtung auch geprüft, welche Berechtigung ein authentifizierter Benutzer hat. So kann es manchen Benutzern erlaubt sein, die Datenschnittstelle zu benutzen, beispielsweise Ärzten oder Servicetechnikern.

Patienten kann es aber zu ihrer eigenen Sicherheit untersagt sein, die Datenschnittstelle zur benutzen. Je nach Berechtigung kann der Datenaustausch über die Datenschnittstelle erlaubt, verhindert oder auch eingeschränkt erlaubt werden. Ein Einschränkung des Datenaustauschs kann beispielsweise bedeuten, dass Patienten über die Datenschnittstelle Zugang zu bestimmten E-Mail Diensten erhalten, jede anderer Datenaustausch aber aus Sicherheitsgründen verhindert wird. Eine solches Erlauben, Verhindern oder Einschränken des Datenaustauschs wird beispielsweise durch eine entsprechende Software realisiert, die im medizintechnischen Gerät gespeichert ist und eine als Mikrokontroller oder Prozessor ausgeführte Steuerelektronik, die den Datenaustausch über die Datenschnittstelle regelt, programmiert.

### Kurze Beschreibung der Figuren

Weitere Einzelheiten und Vorteile der Erfindung werden anhand von in den Zeichnungen dargestellten Ausführungsbeispielen näher beschrieben. Es zeigen:
Die Figur 1 eine schematische Darstellung eines als Hämodialysegerät ausgeführten medizintechnischen Geräts im Einklang mit der Lehre der vorliegenden Erfindung und
die Figur 2 eine schematische Darstellung einer Erkennungsvorrichtung im Einklang mit der Lehre der vorliegenden Erfindung.

### Ausführliche Beschreibung der Figuren

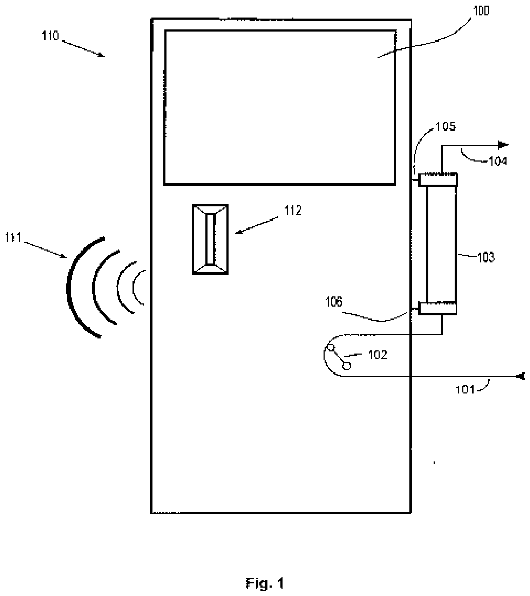

In Figur 1 ist schematisch ein als Hämodialysegerät ausgeführtes Blutbehandlungsgerät als Ausführungsform eines medizintechnischen Geräts im Einklang mit der Lehre der vorliegenden Erfindung dargestellt. Das Hämodialysegerät 110 zeigt andeutungsweise Teile eines extrakorporalen Blutkreislaufs mit einer arteriellen Blutleitung 101, die Blut eines Patienten (nicht dargestellt) ableitet. Die Blutpumpe 102 fördert das Blut durch einen Dialysefilter 103, der mit einer semipermeablen Membran ausgestattet ist, die den extrakorporalen Blutkreislauf von einem Dialysatkreislauf semipermeabel trennt. Über die venöse Leitung 104 wird das behandelte Blut dem Patienten zurückgegeben. Über die Dialysatleitungen 105 und 106 wird Dialysat durch den Dialysefilter 103 gepumpt, wo es über die semipermeable Membran des Dialysefilters 103 zu einem diffusiven Stoffaustausch mit dem Blut des Patienten kommt. Wird zusätzlich ein Druckgradient von der Blutseite des Dialysefilters zur Dialysatseite des Patienten aufgebaut, wird Plasmawasser aus dem Blut in das Dialysat abgepresst. Das Blut des Patienten kann so entwässert werden. Das Dialysat wird in dem Hämodialysegerät 110 hergestellt und nach Gebrauch verworfen. Die Blutpumpe 103 kann im Einklang mit der Lehre der vorliegenden Erfindung als Zentrifugalpumpe oder als Schlauchrollenpumpe ausgeführt sein.

Das Dialysegerät weist zur kombinierten Ein- und Ausgabe von Information eine Touchscreendisplay 100 auf. Weiterhin ist das Dialysegerät im Einklang mit der Lehre der vorliegenden Erfindung mit einer Schnittstelle 111 zum Datenaustausch mit einem Netzwerk ausgestattet. Diese befindet sich im Inneren des Dialysegeräts und ist in Figur 1 exemplarisch als WLAN Schnittstelle ausgeführt und durch konzentrische Kreisabschnitte symbolisiert.

Weiterhin weist das Dialysegerät eine als Kartenlesegerät ausgeführte Authentifiziereinrichtung 112 auf. Bei einem solchermaßen ausgestatteten Dialysegerät ist die in Figur 1 nicht dargestellte Steuerelektronik im Einklang mit der Lehre der vorliegenden Erfindung dazu eingerichtet, die Schnittstelle 111 zum Datenaustausch mit einem Netzwerk zu aktivieren.

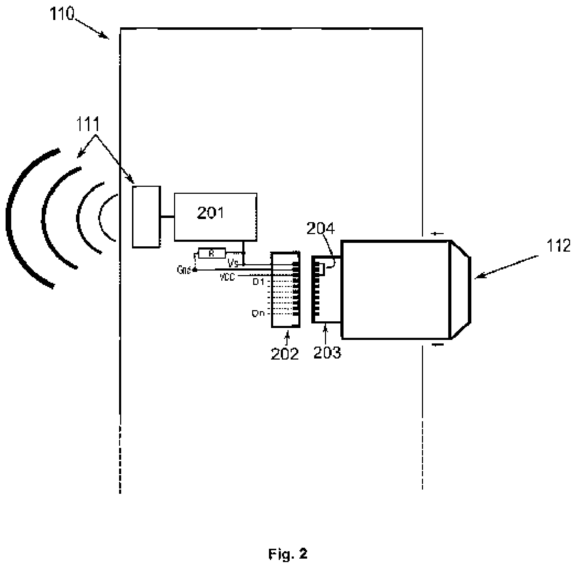

Die Figur 2 zeigt eine vereinfachte Schnittbilddarstellung des Dialysegeräts 110 aus der Figur 1 mit einer Ausführungsform der Steuerelektronik in einem Dialysegerät im Einklang mit der Lehre der vorliegenden Erfindung.

Die Steuerelektronik weist ein Steuergerät 201 auf, welches als Mikrokontroller ausgeführt sein kann. Die Steuerelektronik weist eine Erkennungseinrichtung auf, die in dem vorliegenden Ausführungsbeispiel aus der Kontaktleiste 202, den Versorgungsleitungen mit den Spannungspotentialen VDD und Gnd, der Signalleitung Vs und dem Widerstand R besteht. Die Erkennungseinrichtung ist derart konfiguriert, dass an der Signalleitung Vs das Potential VDD anliegt, wenn das Dialysegerät 110 mit einer Authentifiziereinrichtung 112 ausgestattet ist. Ist das Dialysegerät 110 nicht mit einer Authentifiziereinrichtung 112 ausgestattet, liegt an der Signalleitung VS das Potential Gnd an.

Hierzu ist die Kontaktleiste 202 vorgesehen, die sich im Innern des Dialysegeräts 110 befindet. Diese Kontaktleiste ist beispielsweise eine Steckkartenverbindung zur Aufnahme von Steckkarten und weist mehrere Kontaktflächen zur Weiterleitung elektrischer Signale auf.

Die Erkennungseinrichtung ist im vorliegenden Beispiel derart konfiguriert, dass an der obersten Kontaktfläche der Kontaktleiste 202 das Signal Vs abgegriffen wird. An der zweiten Kontaktfläche von oben ist das Versorgungspotential Gnd angeschlossen, und an der dritten Kontaktfläche ist das Versorgungspotential VDD angeschlossen.

Die Kontaktleiste 202 weist noch ein Vielzahl weiterer Kontaktflächen D1 bis Dn auf, die zur Weiterleitung elektrischer Signale, beispielsweise digitaler Daten vorgesehen sein können.

Das Kartenlesegerät 112 weist im vorliegenden Beispiel an seiner Rückseite eine Steckkarte 203 mit Kontaktflächen auf, die das Gegenstück zur Kontaktleiste 202 bildet. Die Steckkarte 203 und die Kontaktleiste 202 können passgenau ineinandergesteckt werden, wobei die jeweils gegenüber liegenden Kontaktflächen elektrisch leitend miteinander verbunden werden. Die Steckkarte 203 weist eine Verbindungsleitung 204 auf, die die oberste Kontaktfläche der Steckkarte 203 mit der dritten Kontaktfläche von oben der Steckkarte 203 elektrisch verbindet.

Bei der Ausrüstung des Dialysegeräts 110 mit dem Kartenlesegerät 112 wird das Karten lesegerät 112 beispielsweise in eine Aussparung des Gehäuses des Dialysegeräts derart montiert, dass die Steckkarte 203 in die Kontakteleiste 202 gesteckt wird, wodurch die gegenüber liegenden Kontaktflächen elektrisch leitend miteinander verbunden werden.

Auf diese Weise kann das Karten lesegerät mit Energie durch die Versorgungspotentiale VDD und Gnd versorgt werden, und die Signale des Kartenlesegeräts können an den Kontaktflächen D1 bis Dn der Steuerelektronik zur Verfügung gestellt werden (in Figur 2 durch die gestrichelten Signalleitungen D1 bis Dn angedeutet).

Durch das Ineinanderstecken der Steckkarte 203 und der Kontaktleiste 202 in Folge der Montage des Kartenlesegeräts 112 wird das Versorgungspotential VDD über die Verbindungsleitung 204 elektrisch mit der Signalleitung Vs verbunden, welche dann das Potential VDD annimmt.

Ist kein Karten lesegerät im Dialysegerät vorhanden, fehlt diese elektrische Verbindung. Die Signalleitung Vs ist in diesem Fall über den Widerstand R mit dem Versorgungspotential Gnd verbunden und nimmt dann das Potential Gnd an.

Die Signalleitung Vs ist mit dem Steuergerät 201 elektrisch verbunden. Das Steuergerät ist derart konfiguriert, das Potential an der Versorgungsleitung Vs zu überprüfen und die Schnittstelle 111 zum Datenaustausch mit einem Netzwerk (hier als WLAN Adapter ausgeführt) zu deaktivieren, wenn das Potential an der Signalleitung Vs gleich Gnd ist und zu aktivieren, wenn das Potential an der Signalleitung Vs gleich VDD ist. Gnd und VDD sind hierbei äquivalent zu den digitalen Signalen "0" und "1".

Das Aktivieren bzw. Deaktivieren kann beispielsweise gleichbedeutend sein mit dem Versorgen der Schnittstelle 111 mit Energie, bzw. mit der Trennung der Schnittstelle 111 von der Energieversorgung.

Eine weitere Ausführungsform sieht vor, dass zusätzlich geprüft wird, welche Berechtigung ein Benutzer des Dialysegeräts hat und abhängig von dieser Überprüfung der Datenaustausch mit einem Netzwerk über die Schnittstelle 111 erlaubt, verhindert oder eingeschränkt erlaubt wird.

Die Berechtigung des Benutzers wird im vorliegenden Beispiel durch Auslesen der der SmardCard im Kartenlesegerät in oben beschriebener Weise in Erfahrung gebracht und dem Steuergerät 201, beispielsweise über die Datenleitungen D1 bis Dn zugeführt (in Figur 2 nicht dargestellt). Das Steuergerät 201 kann beispielsweise durch entsprechende Programmierung derart konfiguriert sein, abhängig von der Berechtigung des Benutzers den Datenaustausch mit einem Netzwerk über die Schnittstelle 111 zu erlauben, zu verhindern oder eingeschränkt zu erlauben.

Das Dialysegerät analog der Figur 2 zeigt nur exemplarisch eine Ausführungsform der Erfindung mit einer als Karten lesegerät ausgeführten Authentifiziereinrichtung 112 und einer exemplarisch konfigurierten Steuerelektronik, welche eine exemplarisch ausgeführte Erkennungseinrichtung aufweist. Vielfältige andere Ausführungen sind im Einklang mit der Lehre der vorliegenden Erfindung möglich.

## Patentansprüche

1. Medizintechnisches Gerät umfassend eine Steuerelektronik und eine Schnittstelle zum Datenaustausch mit einem Netzwerk, wobei das medizintechnische Gerät zur Ausrüstung mit einer Authentifiziereinrichtung eingerichtet ist, und wobei die Steuerelektronik dazu eingerichtet ist, die Schnittstelle zum Datenaustausch mit einem Netzwerk zu deaktivieren, wenn das medizintechnische Gerät nicht mit einer Authentifiziereinrichtung ausgerüstet ist und die Schnittstelle zum Datenaustausch mit einem Netzwerk zu aktivieren, wenn das medizintechnische Gerät mit einer Authentifiziereinrichtung ausgerüstet ist.

2. Medizintechnisches Gerät nach Anspruch 1, wobei die Authentifiziereinrichtung ein Kartenlesegerät oder ein Fingerabdruckscanner oder ein Irisscanner ist.

3. Medizintechnisches Gerät nach einem der vorigen Ansprüche, wobei die Steuerelektronik eine Erkennungseinrichtung aufweist und die Erkennungseinrichtung so konfiguriert ist, dass die Ausrüstung des medizintechnischen Geräts mit einer Authentifiziereinrichtung anhand der Überprüfung zumindest eines Signals erkannt wird, welches durch die Ausrüstung mit der Authentifiziereinrichtung erzeugt wird.

4. Medizintechnisches Gerät nach einem der vorigen Ansprüche, wobei die Steuerelektronik dazu eingerichtet ist, eine durch die Authentifiziereinrichtung bestimmte Berechtigung eines Benutzers zu prüfen und aufgrund der Prüfung den Datenaustausch mit einem Netzwerk zu erlauben oder zu verhindern oder eingeschränkt zu erlauben.

5. Medizintechnisches Gerät nach einem der vorigen Ansprüche, wobei die Schnittstelle zum Datenaustausch mit einem Netzwerk kabelgebunden, beispielsweise als RJ45 Datenschnittstelle ausgeführt ist, oder kabellos, beispielsweise als WLAN oder Bluetooth Schnittstelle ausgeführt ist.

6. Medizintechnisches Gerät nach einem der vorigen Ansprüche, wobei das medizintechnische Gerät ein Gerät zur Blutbehandlung ist, insbesondere ein Dialysegerät.

7. System umfassend ein medizintechnisches Gerät nach einem der Ansprüche 1 bis 6 und ein an die Schnittstelle zum Datenaustausch angeschlossenes Netzwerk und ein an das Netzwerk zum Datenaustausch mit dem medizintechnischen Gerät angeschlossenes entferntes Gerät.

8. System nach Anspruch 7, wobei das entfernte Gerät ein Server ist.

9. Verfahren zum Datenaustausch zwischen einem medizintechnischen Gerät umfassend eine Schnittstelle zum Datenaustausch mit einem Netzwerk, wobei das medizintechnische Gerät zur Ausrüstung mit einer Authentifiziereinrichtung eingerichtet ist, umfassend die Schritte:
Deaktivieren der Schnittstelle zum Datenaustausch mit einem Netzwerk, wenn das medizintechnische Gerät nicht mit einer Authentifiziereinrichtung ausgerüstet ist und
Aktivieren der Schnittstelle zum Datenaustausch mit einem Netzwerk, wenn das medizintechnische Gerät mit einer Authentifiziereinrichtung ausgerüstet ist.

10. Verfahren nach Anspruch 9, wobei die Authentifiziereinrichtung ein Kartenlesegerät oder ein Fingerabdruckscanner oder ein Irisscanner ist.

11. Verfahren nach einem der Ansprüche 9 oder 10, wobei eine Erkennungseinrichtung die Ausrüstung des medizintechnischen Geräts mit einer Authentifiziereinrichtung anhand der Überprüfung zumindest eines Signals erkennt, welches durch die Ausrüstung mit der Authentifiziereinrichtung erzeugt wird.

12. Verfahren nach einem der Ansprüche 9 bis 11, wobei eine durch die Authentifiziereinrichtung bestimmte Berechtigung eines Benutzers geprüft wird und aufgrund der Prüfung der Datenaustausch mit einem Netzwerk erlaubt oder verhindert oder eingeschränkt erlaubt wird.

13. Verfahren nach einem der vorigen Ansprüche 9 bis 12, wobei die Schnittstelle zum Datenaustausch mit einem Netzwerk kabelgebunden, beispielsweise als RJ45 Datenschnittstelle ausgeführt ist, oder kabellos, beispielsweise als WLAN oder Bluetooth Schnittstelle ausgeführt ist.

14. Verfahren nach einem der vorigen Ansprüche 9 bis 13, wobei das medizintechnische Gerät ein Gerät zur Blutbehandlung ist, insbesondere ein Dialysegerät.

## Claims

1. A medical technical device, comprising an electronic control system and an interface for data exchange with a network,
wherein the medical technical device is established for being furnished with an authentication unit, and
wherein the electronic control system is established to deactivate the interface for data exchange with a network when the medical technical device is not equipped with an authentication unit and to activate the interface for data exchange with a network when the medical device is equipped with an authentication unit.

2. The medical technical device according to claim 1,
wherein the authentication unit is a card reader or a fingerprint scanner or an iris scanner.

3. The medical technical device according to any one of the preceding claims,
wherein the electronic control system has a recognition device, and the recognition device is configured so that the equipment of the medical technical device having an authentication unit is recognized on the basis of the verification of at least one signal generated by the equipment having the authentication unit.

4. The medical technical device according to any one of the preceding claims,
wherein the electronic control system is established to check an authorization of a user determined by the authentication unit and to allow or prevent or allow to a limited extent data exchange with a network on the basis of this check.

5. The medical technical device according to any one of the preceding claims,
wherein the interface for data exchange with a network is embodied as a hardwired interface, for example, as an RJ45 data interface, or as a wireless interface, for example, as a WLAN or Bluetooth interface.

6. The medical technical device according to any one of the preceding claims,
wherein the medical technical device is a device for treatment of blood, in particular a dialysis machine.

7. A system comprising a medical technical device according to any one of claims 1 to 6 and a network connected to the interface for data exchange and a remote device connected to the network for data exchange having the medical technical device.

8. The system according to claim 7,
wherein the remote device is a server.

9. A method for data exchange between a medical technical device, comprising an interface for data exchange with a network, wherein the medical technical device is established for being furnished with an authentication unit, comprising the following steps:
deactivation of the interface for data exchange with a network when the medical technical device is not equipped with an authentication unit, and
activation of the interface for data exchange with a network when the medical technical device is equipped with an authentication unit.

10. The method according to claim 9,
wherein the authentication unit is a card reader or a fingerprint scanner or an iris scanner.

11. The method according to any one of claims 9 or 10,
wherein a recognition unit recognizes the equipment of the medical technical device having an authentication unit on the basis of the verification of at least one signal generated by equipment having the authenticcation unit.

12. The method according to any one of claims 9 or 11,
wherein the authorization of a user determined by the authentication unit is checked and then allowed or prevented or allowed to a restricted extent on the basis of the check of the data exchange with a network.

13. The method according to any one of preceding claims 9 to 12,
wherein the interface is hardwired for data exchange with a network, for example, being designed as an RJ45 data interface, or is wireless, for example, being designed as a WLAN or Bluetooth interface.

14. The method according to any one of preceding claims 9 to 13,
wherein the medical technical device is a device for treatment of blood, in particular a dialysis machine.

## Revendications

1. Appareil technique médical comprenant un système électronique de commande est une interface d'échange de données avec un réseau, l'appareil technique médical étant conçu pour être équipé d'un dispositif d'authentification et le système électronique de commande étant conçu pour désactiver l'interface d'échange de données avec un réseau lorsque l'appareil technique médical n'est pas équipé du dispositif d'authentification et pour activer l'interface d'échange de données avec un réseau lorsque l'appareil médical est équipé du dispositif d'authentification.

2. Appareil technique médical selon la revendication 1, dans lequel le dispositif d'authentification est un lecteur de cartes ou un scanner d'empreintes digitales ou un scanner d'iris.

3. Appareil technique médical selon une des revendications précédentes, dans lequel le système électronique de commande présente un dispositif de détection et le dispositif de détection est configuré de manière à ce que l'équipement de l'appareil technique médical avec un dispositif d'authentification soit détecté à partir du contrôle d'au moins un signal qui est généré par l'équipement comportant le dispositif d'identification.

4. Appareil technique médical selon une des revendications précédentes, dans lequel le système électronique de commande est conçu pour contrôler une autorisation d'un utilisateur définie par le dispositif d'authentification et pour autoriser ou refuser ou autoriser de manière restreinte l'échange de données avec un réseau sur la base du contrôle.

5. Appareil technique médical selon une des revendications précédentes, dans lequel l'interface d'échange de données avec un réseau est réalisée avec une liaison câblée, par exemple sous forme d'une interface de données RJ 45, ou sans câble, par exemple sous forme d'une face WLAN ou Bluetooth.

6. Appareil technique médical selon une des revendications précédentes, dans lequel l'appareil médical est un appareil de traitement du sang, en particulier un appareil de dialyse.

7. Système comprenant un appareil technique médical selon une des revendications 1 à 6 et un réseau raccordé à l'interface d'échange de données et un appareil supprimé raccordé au réseau pour l'échange de données avec l'appareil technique médical.

8. Systèmes selon la revendication 7, dans lequel l'appareil supprimé est un serveur.

9. Procédé d'échange de données entre un appareil technique médical comprenant une interface d'échange de données avec un réseau, l'appareil technique médical étant conçu pour être équipé d'un dispositif d'authentification, comprenant les étapes suivantes :
désactivation de l'interface d'échange de données avec un réseau lorsque l'appareil technique médical n'est pas équipé d'un dispositif d'authentification et
activation de l'interface d'échange de données avec un réseau lorsque l'appareil technique médical est équipé d'un dispositif d'authentification.

10. Procédé selon la revendication 9, dans lequel le dispositif d'authentification est un lecteur de cartes ou un scanner d'empreintes digitales ou un scanner d'iris.

11. Procédé selon une des revendications 9 ou 10, dans lequel un dispositif de détection détecte l'équipement de l'appareil technique médical avec un dispositif d'authentification à partir du contrôle d'au moins un signal qui est généré par l'équipement comportant le dispositif d'authentification.

12. Procédé selon une des revendications 9 à 11, dans lequel une autorisation d'un utilisateur définie par le dispositif d'authentification est contrôlée et l'échange de données avec un réseau est autorisé ou refusé ou autorisé de manière restreinte sur la base du contrôle.

13. Procédé selon une des revendications précédentes 9 à 12, dans lequel l'échange de données avec un réseau a lieu par liaison câblée, par exemple sous forme d'interface de données RJ 45, ou sans câble, par exemple sous forme d'interface WLAN ou Bluetooth.

14. Procédé selon une des revendications 9 à 13, dans lequel l'appareil technique médical est un appareil de traitement du sang, en particulier un appareil de dialyse.
